# EUROPEAN PATENT APPLICATION

(11) **EP 2 811 019 A1**
(43) Date of publication of application: **10.12.2014**
(21) Application number: 13743774.5
(22) Date of filing: 23.01.2013
(51) Int. Cl.: C12N 15/09, C12Q 1/68

(54) **METHOD FOR CORRECTING DETECTION SIGNAL IN ISOTHERMAL NUCLEIC ACID AMPLIFICATION REACTION**

(30) Priority: 31.01.2012 JP 2012018277
(71) Applicant: FUJIREBIO INC., Shinjuku-ku, Tokyo 163-0410 (JP)
(72) Inventor: MATSUNO, Tatsuki, Tokyo 163-0410 (JP)
(74) Representative: Hoffmann Eitle
(86) International application number: PCT/JP2013/051276
(87) International publication number: WO 2013/115029

(57) **Abstract**

The present invention provides a method for stably correcting a detection signal in an isothermal nucleic acid amplification reaction, particularly an isothermal nucleic acid amplification reaction performed at low temperature.

Specifically, the present invention provides a method for detecting a target nucleic acid in an isothermal nucleic acid amplification reaction, comprising the following (1) and (2):
(1) subjecting a nucleic acid sample to an amplification reaction of a target nucleic acid under an isothermal condition in the presence of a nucleic acid probe for correction and a nucleic acid probe for detection of the target nucleic acid; and
(2) correcting a detection signal due to the nucleic acid probe for detection with a detection signal due to the nucleic acid probe for correction in the amplification reaction.

## Description

### TECHNICAL FIELD

The present invention relates to a method for correcting a detection signal in an isothermal nucleic acid amplification reaction, and the like.

### BACKGROUND ART

Generally, an internal reference fluorescent substance such as ROX (6-carboxy-X-rhodamine) is used to stabilize a baseline of a fluorescence signal detected in a real-time nucleic acid amplification assay such as real-time PCR. Those disclosed in Patent Literature 1 are also known as the internal reference fluorescent substance.

### PRIOR ART REFERENCES

### PATENT LITERATURES

Patent Literature 1: WO97/046708

### DISCLOSURE OF INVENTION

### PROBLEM TO BE SOLVED BY THE INVENTION

However, a baseline is not stabilized in an isothermal nucleic acid amplification reaction, particularly an isothermal nucleic acid amplification reaction performed at low temperature. Therefore, it is difficult to stably correct a detection signal from a subject to be measured.

### MEANS FOR SOLVING PROBLEM

The present inventors thought that a reason why the baseline of the fluorescence signal was not stabilized in the isothermal nucleic acid amplification reaction, particularly the isothermal nucleic acid amplification reaction performed at low temperature came from a fact that a nucleic acid probe for detection and an internal reference fluorescent substance often exhibited a different behavior. As a result of an extensive study under such a hypothesis, the present inventors have found that the baseline of the fluorescence signal in the isothermal nucleic acid amplification reaction can be stabilized by use of a nucleic acid probe for correction that can mimic a behavior of the nucleic acid probe for detection and therefore a detection signal from a subject to be measured can be corrected stably, and completed the present invention.

Accordingly, the present invention is as follows.
[1] A method for detecting a target nucleic acid in an isothermal nucleic acid amplification reaction, comprising the following (1) and (2):
   (1) subjecting a nucleic acid sample to an amplification reaction of the target nucleic acid under an isothermal condition in the presence of a nucleic acid probe for correction and a nucleic acid probe for detection of the target nucleic acid; and
   (2) correcting a detection signal due to the nucleic acid probe for detection with a detection signal due to the nucleic acid probe for correction in the amplification reaction.
[2] The method of [1], wherein the nucleic acid probe for correction comprises a single strand polynucleotide portion comprising a nucleotide sequence that is not complementary to a nucleotide sequence of the target nucleic acid, and a detection signal generation portion,
   wherein the nucleic acid probe for detection comprises a single strand polynucleotide portion comprising a nucleotide sequence that is complementary to the nucleotide sequence of the target nucleic acid, and a detection signal generation portion,
   wherein the detection signal generation portion of the nucleic acid probe for correction and the detection signal generation portion of the nucleic acid probe for detection are different from each other.
[3] The method of [2], wherein the nucleic acid probe for correction comprises a single strand polynucleotide portion consisting of 8 to 50 nucleotide residues,
   wherein the nucleic acid probe for detection comprises a single strand polynucleotide portion consisting of 8 to 50 nucleotide residues.
[4] The method of [3], wherein a difference in length between the nucleic acid probe for correction and the nucleic acid probe for detection is within the range of 9 nucleotide residues.
[5] The method of [4], wherein the difference in length between the nucleic acid probe for correction and the nucleic acid probe for detection is within the range of 3 nucleotide residues.
[6] The method of any of [2] to [5], wherein a nucleotide sequence of the single strand polynucleotide portion in the nucleic acid probe for correction exhibits 50% or less identity to a nucleotide sequence of the single strand polynucleotide portion in the nucleic acid probe for detection.
[7] The method of [2], wherein the detection signal generation portion in the nucleic acid probe for correction and the detection signal generation portion in the nucleic acid probe for detection are different fluorescent substances.
[8] The method of [7], wherein the fluorescent substance has a nature that fluorescence is quenched by formation of a double strand nucleic acid structure.
[9] The method of [8], which is either the following (I) or (II):
   (I) the nucleic acid probe for correction has a cytosine-containing nucleotide linked to a first fluorescent substance in which a fluorescence signal reduces when positioned adjacent to a guanine base, at a 3' end of the single strand polynucleotide portion thereof, and the nucleic acid probe for detection has a cytosine-containing nucleotide linked to a second fluorescent substance in which a fluorescence signal reduces when positioned adjacent to a guanine base, at a 3' end of the single strand polynucleotide portion thereof; or
   (II) the nucleic acid probe for correction has a cytosine-containing nucleotide linked to a first fluorescent substance in which a fluorescence signal reduces when positioned adjacent to a guanine base, at a 5' end of the single strand polynucleotide portion thereof, and the nucleic acid probe for detection has a cytosine-containing nucleotide linked to a second fluorescent substance in which a fluorescence signal reduces when positioned adjacent to a guanine base, at a 5' end of the single strand polynucleotide portion thereof.
[10] The method of any of [1] to [9], wherein the target nucleic acid is amplified under an isothermal condition at lower than 50°C.
[11] The method of any of [1] to [10], wherein the isothermal nucleic acid amplification reaction is a method using a recombinase.
[12] A kit comprising a nucleic acid probe for correction and a nucleic acid probe for detection of a target nucleic acid.
[13] The kit of [12], further comprising a polymerase.
[14] The Kit of [12] or [13], further comprising a recombinase.

### EFFECT OF THE INVENTION

The present invention can stably correct a detection signal from a subject to be measured in an isothermal nucleic acid amplification reaction, particularly an isothermal nucleic acid amplification reaction performed at low temperature by use of a nucleic acid probe for correction. Therefore, the present invention is useful for qualitative and quantitative measurement of the subject to be measured by the isothermal nucleic acid amplification reaction. Specifically, the method of the present invention is excellent in accuracy in detection of the target nucleic acid in the isothermal nucleic acid amplification reaction. In addition, according to the kit of the present invention, the detection of the target nucleic acid in the isothermal nucleic acid amplification reaction can be carried out conveniently.

### BRIEF DESCRIPTION OF DRAWINGS

FIG. 1 is a view illustrating correction of a fluorescence intensity change rate with a nucleic acid probe for correction (Reference 1). A boxed numerical value (%) denotes the fluorescence intensity change rate one minute after starting a reaction, and a boxed time period (minutes) denotes a time period required for stabilizing the fluorescence intensity change rate (time period to reach a baseline). DW: Distilled water. The same shall apply hereinafter.
FIG. 2 is a view illustrating correction of a fluorescence intensity change rate with ROX reference dye.
FIG. 3 is a graph showing correction of a fluorescence intensity change rate with a nucleic acid probe for correction (Reference 2).
FIG. 4 is a graph showing correction of a fluorescence intensity change rate with a nucleic acid probe for correction (Reference 3).
FIG. 5 is a graph showing correction of a fluorescence intensity change rate with a nucleic acid probe for correction (Reference 4).
FIG. 6 is a graph showing correction of a fluorescence intensity change rate with a nucleic acid probe for correction (Reference 5).
FIG. 7 is a graph showing correction of a fluorescence intensity change rate with a nucleic acid probe for correction (Reference 6).
FIG. 8 is a graph showing correction of a fluorescence intensity change rate with a nucleic acid probe for correction (Reference 7).

### EMBODIMENTS FOR CARRYING OUT THE INVENTION

The present invention provides a method for correcting a detection signal in an isothermal nucleic acid amplification reaction.

Any method that can amplify a target nucleic acid under an isothermal condition is used as the isothermal nucleic acid amplification reaction. Examples of such a method may include a method mediated by transcription, a method using a nicking enzyme (e.g., a restriction enzyme, ribonuclease H), a method utilizing a series of DNA synthesis through a loop structure, a method utilizing a rolling circle type of DNA replication reaction, a method using a helicase, and a method using a recombinase. Example of the method mediated by the transcription may include TMA (Transcription Mediated Amplification) method and NASBA (Nucleic Acid Sequence-Based Amplification) method. Examples of the method using the nicking enzyme such as the restriction enzyme or the ribonuclease H may include SDA (Strand Displacement Amplification) method and ICAN (Isothermal and Chimeric Primer-initiated Amplification of Nucleic Acids) method. Examples of the method utilizing a series of DNA synthesis through the loop structure may include LAMP (Loop-mediated Isothermal Amplification) method and SMAP (Smart Amplification Process) method. Examples of the method utilizing the rolling circle type of DNA replication reaction may include RCA (Rolling Circle Amplification) method. Examples of the method using the helicase may include HAD (Helicase-Dependent Amplification) method. Example of the method using the recombinase may include RPA (Recombinase Polymerase Amplification) method and SIBA (Strand Invasion Based Amplification) method.

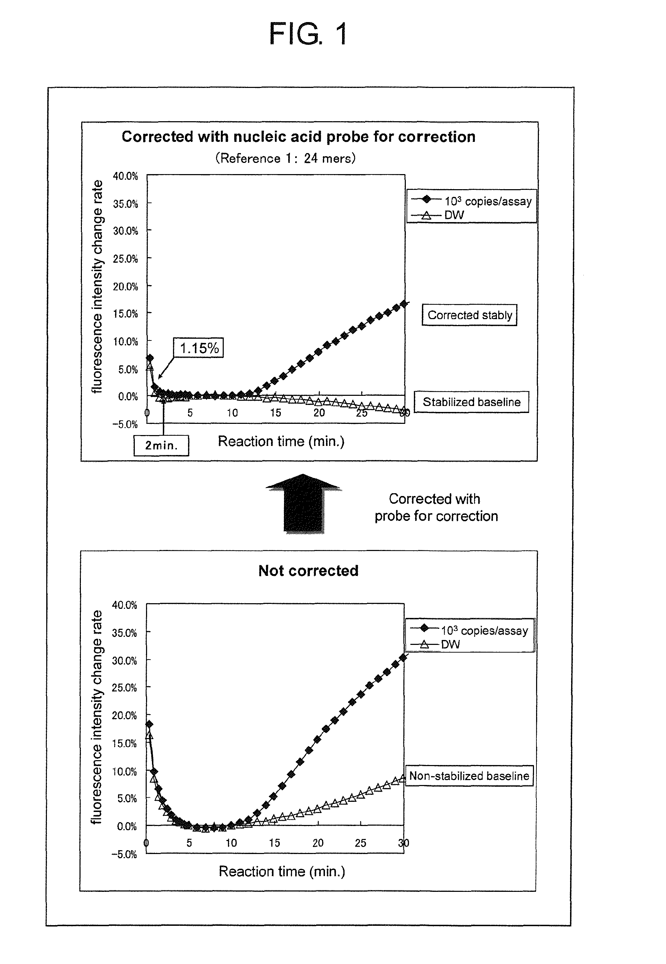

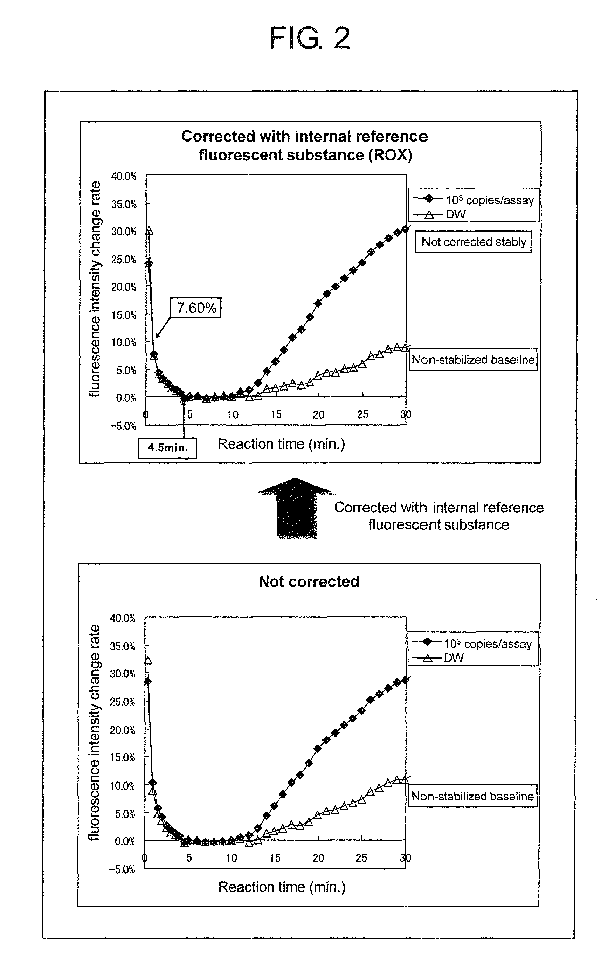

The isothermal nucleic acid amplification reaction is mainly classified into an isothermal nucleic acid amplification reaction performed under a condition at medium or high temperature of 50°C or higher (e.g., 50°C or higher and lower than 70°C) and an isothermal nucleic acid amplification reaction performed under a condition at low temperature of lower than 50°C (e.g., 25°C or higher and lower than 50°C). Conventionally, in the isothermal nucleic acid amplification reaction, particularly the isothermal nucleic acid amplification reaction performed at low temperature, a nucleic acid probe for detection and an internal reference fluorescent substance often exhibit different behavior, and thus it was difficult to stabilize baseline of a detection signal (see Reference Example 1 and DW in FIG. 2). On the other hand, in the method of the present invention, the nucleic acid probe for correction can exhibit a behavior that is similar to that of a nucleic acid probe for detection even under a condition at low temperature, and thus it can be accomplished to stabilize baseline of a detection signal and stably correct the detection signal from a subject to be measured (see, e.g., Example 1 and FIG. 1). Specifically, the method of the present invention can accomplish the stabilization of the baseline of the detection signal and the stable correction of the detection signal from the subject to be measured even when performed under the condition at low temperature of lower than 50°C, preferably lower than 45°C and more preferably lower than 42°C. Among the aforementioned isothermal nucleic acid amplification reactions, the method mediated by the transcription, the method using the nicking enzyme such as the restriction enzyme or the ribonuclease H, the method utilizing the rolling circle type of DNA replication reaction, the method using the helicase, and the method using the recombinase can be carried out under such a low temperature condition. Therefore, the method of the present invention can be suitably carried out even under such a low temperature condition.

The present invention comprises the following (1) and (2) :
(1) subjecting a nucleic acid sample to an amplification reaction of a target nucleic acid under an isothermal condition in the presence of a nucleic acid probe for correction and a nucleic acid probe for detection of the target nucleic acid; and
(2) correcting the detection signal due to the nucleic acid probe for detection with the detection signal due to the nucleic acid probe for correction in the amplification reaction.

The nucleic acid probe for detection of a target nucleic acid is a nucleic acid probe that generates a detectable signal by specifically binding to the target nucleic acid. The nucleic acid probe for detection of a target nucleic acid comprises a single strand polynucleotide portion comprising a nucleotide sequence that is complementary to a nucleotide sequence of the target nucleic acid, and a detection signal generation portion.

The nucleic acid probe for detection is designed so that its single strand polynucleotide portion complementarily binds to the target nucleic acid. The single strand polynucleotide portion in the nucleic acid probe for detection is composed of nucleotides having nucleobases selected from the group consisting of adenine (A), guanine (G), cytosine (C), uracil (U) and thymine (T) and modified nucleobases thereof (e.g., inosine, uridine, 3-nitropyrrole, 5-nitroindole, pyrimidine derivatives). A person skilled in the art can appropriately select nucleotides having the modified nucleobases that can constitute the single strand polynucleotide portion in the nucleic acid probe for detection. The single strand polynucleotide portion in the nucleic acid probe for detection may be composed only of the nucleotides having nucleobases selected from the group consisting of adenine (A), guanine (G), cytosine (C), uracil (U) and thymine (T) that are commonly used as the nucleotides that constitute the nucleic acid probe. The single strand polynucleotide portion may be a DNA or RNA molecule or a hybrid molecule of DNA and RNA.

The single strand polynucleotide portion in the nucleic acid probe for detection may be defined in terms of nucleotide sequence percent identity. Specifically, a nucleotide sequence of the single strand polynucleotide portion in the nucleic acid probe for detection exhibits a significant nucleotide sequence percent identity to a nucleotide sequence of a certain region in a complementary strand of the target nucleic acid to the extent that the single strand polynucleotide portion can be complementarily bound to the target nucleic acid. Such a significant nucleotide sequence percent identity that is calculated between the nucleotide sequence of the single strand polynucleotide portion in the nucleic acid probe for detection and the nucleotide sequence of the above certain region is, for example 90% or more, preferably 92% or more, more preferably 94% or more, still more preferably 95% or more, 96% or more, 97% or more, 98% or more, or 99% or more, and particularly preferably 100%. In this case, the nucleotide sequence percent identity is calculated based on the length of the nucleotide sequence of the single strand polynucleotide portion in the nucleic acid probe for detection. The nucleotide sequence percent identity can be determined by a method well-known in the art, and for example, a program available on internet, in which two nucleotide sequences can be compared, may be utilized with default setting. For example, the nucleotide sequence percent identity may be calculated by algorithm of Huang and Miller (Adv. Appl. Math. (1991) 12: 337-357). The calculation by the algorithm of Huang and Miller can be performed easily by utilizing William Pearson's lalign program published at http://www.ch.embnet.org/software/LALIGN_form.html with default setting (with the alignment method is made global).

The length of the single strand polynucleotide portion in the nucleic acid probe for detection is not particularly limited as long as it can be complementarily bound to the target nucleic acid under a condition (e.g., temperature condition) employed in the method of the present invention, and can be appropriately determined depending on a type of the isothermal nucleic acid amplification reaction employed in the method of the present invention. In general, the length of the single strand polynucleotide portion is 8 to 50 nucleotides in length, preferably 10 to 40 nucleotides in length, and more preferably 12 to 40 nucleotides in length. The length of the single strand polynucleotide portion may be determined from another perspective. For example, when the method of the present invention is performed by the method using the recombinase (e.g., RPA method), the length of the single strand polynucleotide portion in the nucleic acid probe for detection is, for example, 20 to 50 nucleotides in length, preferably 20 to 40 nucleotides in length, and more preferably 30 to 40 nucleotides in length in terms of recognition of the single strand polynucleotide portion by the recombinase. When the method of the present invention is performed by the method using the recombinase (e.g., RPA method), the target nucleic acid can be detected specifically because the single strand polynucleotide portion in the nucleic acid probe for detection is recognized by the recombinase to be complementarily bound to the target nucleic acid.

The detection signal generation portion in the nucleic acid probe for detection is composed of a substance capable of generating a detectable signal, and enables to detect the target nucleic acid complementarily bound to the single strand polynucleotide portion in the nucleic acid probe for detection. Examples of the detection signal generation portion may include fluorescent substances.

When the detection signal generation portion is the fluorescent substance, the nucleic acid probe for detection may be a probe taking advantage of a fluorescence quenching phenomenon by a nucleobase. Such a nucleic acid probe for detection comprises the above single strand polynucleotide portion and the fluorescent substance having a nature that fluorescence is quenched by formation of a double strand nucleic acid structure. Examples of the fluorescent substance having the nature that the fluorescence is quenched by formation of the double strand nucleic acid structure may include fluorescent substances in which a fluorescence signal reduces when positioned adjacent to the guanine base. Examples of the fluorescent substance in which the fluorescence signal reduces when positioned adjacent to the guanine base may include Pacific blue (2-oxo-6,8-difluoro-7-hydroxy-2H-1-benzopyrane-3-carboxylic acid), BODIPY FL (N-[2-(iodoacetylamino)ethyl]-5,7-dimethyl-4,4-difluoro-3a-azonia-4-bora(IV)-4H-4a-aza-s-indacene-3-propaneamide), 5-CR6G (5-carboxyrhodamine), TAMRA (6-tetramethylrhodamine-5(6)-carboxamide)hexanoate, 6-JOE (6-carboxy-4',5'-dichloro-2',7'-dimethoxyfluorescein), Alexa 488 (trademark), Alexa 532 (trademark), BODIPY TMR, and BODIPY FL/C3.

When the detection signal generation portion is the fluorescent substance, the nucleic acid probe for detection may also be a probe that does not emit fluorescence in an ordinary state. Such a nucleic acid probe for detection further comprises a quencher in addition to the above single strand polynucleotide portion and the fluorescent substance, and emits fluorescence when the fluorescent substance is separated from the quencher in distance. Examples of such a probe may include a hydrolysis probe and a stem-loop structured probe. In the hydrolysis probe, although the fluorescence is quenched while the probe is complementarily bound to the target nucleic acid to form a double strand structure, when the probe is decomposed by an enzyme having an exonuclease activity, the quenching effect is removed and the probe emits fluorescence. The stem-loop structured probe has a self-hairpin structure, and removes its quenching effect and emits fluorescence when the probe is stretched from the hairpin structure upon being complementarily bound to the target nucleic acid. Examples of the fluorescent substance to be used for such a nucleic acid probe for detection may include 6-FAM (6-carboxyfluorescein), TET (tetrachloro-6-carboxyfluorescein), HEX (hexachlorofluorescein), 6-JOE (6-carboxy-4',5'-dichloro-2',7'-dimethoxyfluorescein), ROX (carboxy-6-rhodamine), and TAMRA ((6-tetramethylrhodamine-5(6)-carboxamide)hexanoate). Examples of the quencher to be used for such a nucleic acid probe for detection may include BHQ-1 ([4-(2-nitro-4-methyl-phenyl)-azo)-yl-((2-methoxy-5-methyl-phenyl)-azo)]-aniline), BHQ-2 (([(4-(1-nitro-phenyl)-azo)-yl-((2,5-dimethoxy-phenyl)-azo)]-aniline), Dabcyl (4-[[4-(dimethylamino)-phenyl]-azo]-benzoic acid), Eclipse (4-[[2-chloro-4-nitro-phenyl]-azo]-aniline), and TAMRA ((6-tetramethylrhodamine-5(6)-carboxamide)hexanoate).

In the method of the present invention, a single target nucleic acid or a plurality (e.g., 2, 3, or 4) of target nucleic acids may be employed. When a plurality of target nucleic acids is employed, the same number of the nucleic acid probes for detection can be used. In this case, each of a plurality of target nucleic acids comprises a different type of the signal generation portion. Multiple analyses for a plurality of target nucleic acids are possible by use of a plurality of nucleic acid probes for detection.

The nucleic acid probe for correction is a nucleic acid probe that does not have an ability to bind to the target nucleic acid and generates a detectable signal. The nucleic acid probe for correction comprises a single strand polynucleotide portion comprising a nucleotide sequence that is not complementary to a nucleotide sequence of a target nucleic acid, and a detection signal generation portion. The nucleic acid probe for correction is designed so as to comprise a detection signal generation portion different from that in the nucleic acid probe for detection, thereby discriminating a detection signal generated from the nucleic acid probe for correction from a detection signal generated from the nucleic acid probe for detection.

The nucleic acid probe for correction is designed so that its single strand polynucleotide portion does not complementarily bind to the target nucleic acid. The single strand polynucleotide portion in the nucleic acid probe for correction is composed of nucleotides having nucleobases selected from the group consisting of adenine (A), guanine (G), cytosine (C), uracil (U) and thymine (T) and modified nucleobases thereof (e.g., inosine, uridine, 3-nitropyrrole, 5-nitroindole, pyrimidine derivatives). A person skilled in the art can appropriately select nucleotides having modified nucleobases that can constitute the single strand polynucleotide portion in the nucleic acid probe for correction. In terms of mimicking structural properties of the single strand polynucleotide portion in the nucleic acid probe for detection as possible, it is preferable that the single strand polynucleotide portion in the nucleic acid probe for correction is composed of the same type of nucleotides as the nucleotides that constitute the single strand polynucleotide portion in the nucleic acid probe for detection. Therefore, the single strand polynucleotide portion in the nucleic acid probe for correction may be composed only of nucleotides having nucleobases selected from the group consisting of adenine (A), guanine (G), cytosine (C) and thymine (T) that are commonly used as the nucleotides that constitute nucleic acid probes. The single strand polynucleotide portion may be a DNA or RNA molecule or a hybrid molecule of DNA and RNA. In terms of mimicking structural properties of the single strand polynucleotide portion in the nucleic acid probe for detection as possible, preferably, the single strand polynucleotide portion in the nucleic acid probe for correction is DNA when the single strand polynucleotide portion in the nucleic acid probe for detection is DNA, or is RNA when the single strand polynucleotide portion in the nucleic acid probe for detection is RNA, or is the hybrid molecule when the single strand polynucleotide portion in the nucleic acid probe for detection is the hybrid molecule.

The single strand polynucleotide portion in the nucleic acid probe for correction may be a homomer composed of the same type of nucleotides or a heteromer composed of plural types of nucleotides. It is preferably the heteromer because the nucleic acid probe for detection is typically composed of a plurality of nucleotides and the nucleic acid probe for correction should mimic the behavior of the nucleic acid probe for detection typically composed of a plurality of nucleotides. When the single strand polynucleotide portion is the heteromer, the single strand polynucleotide portion has at least two types of the nucleotides and preferably 3 or 4 types of the nucleotides among the aforementioned nucleotides.

The length of the single strand polynucleotide portion in the nucleic acid probe for correction is 8 to 50 nucleotides in length, preferably 10 to 40 nucleotides in length, and more preferably 12 to 40 nucleotides in length. The length of the single strand polynucleotide portion in the nucleic acid probe for correction is not particularly limited, and may be defined in relation to the length of the single strand polynucleotide portion in the nucleic acid probe for detection. The difference in length between the single strand polynucleotide portion in the nucleic acid probe for correction and the single strand polynucleotide portion in the nucleic acid probe for detection may be, for example, within the range of 9 nucleotide residues, more preferably within the range of 6 nucleotide residues, and still more preferably within the range of 3 nucleotide residues.

The single strand polynucleotide portion in the nucleic acid probe for correction may have a similar GC content to that in the single strand polynucleotide portion in the nucleic acid probe for detection. For example, the GC content (%) in the single strand polynucleotide portion in the nucleic acid probe for correction may be within the range of ±10%, ±7%, ±5% or ±3% relative to the GC content (%) in the single strand polynucleotide portion in the nucleic acid probe for detection.

The single strand polynucleotide portion in the nucleic acid probe for correction may have a similar melting point (Tm value) to that of the single strand polynucleotide portion in the nucleic acid probe for detection. For example, the melting point (°C) of the single strand polynucleotide portion in the nucleic acid probe for correction may be within the range of ±10°C, ±7°C, ±5°C or ±3°C relative to the melting point (°C) in the single strand polynucleotide portion in the nucleic acid probe for detection. The melting point can be appropriately determined by a method well-known in the art, and for example, may be determined using commercially available software.

The single strand polynucleotide portion in the nucleic acid probe for correction may be defined in terms of percent identity to a nucleotide sequence of any region in the complementary strand of the target nucleic acid. Specifically, the nucleotide sequence of the single strand polynucleotide portion in the nucleic acid probe for correction exhibits an insignificant nucleotide sequence percent identity to the nucleotide sequence of any region in the complementary strand of the target nucleic acid to the extent that it cannot complementarily bind to the target nucleic acid. Such an insignificant nucleotide sequence percent identity calculated between the nucleotide sequence of the single strand polynucleotide portion in the nucleic acid probe for correction and the nucleotide sequence of any region mentioned above corresponds to nucleotide sequence percent identity of, for example, 60% or less, 55% or less, 50% or less, 45% or less, 40% or less, 35% or less, or 30% or less. In this case, the nucleotide sequence percent identity is calculated based on the length of the nucleotide sequence of the single strand polynucleotide portion in the nucleic acid probe for correction. The nucleotide sequence percent identity can be determined by a method well-known in the art, and for example, may be determined as described above.

The single strand polynucleotide portion in the nucleic acid probe for correction may be defined in terms of percent identity to the nucleotide sequence of the single strand polynucleotide portion in the nucleic acid probe for detection. Specifically, the nucleotide sequence of the single strand polynucleotide portion in the nucleic acid probe for correction exhibits, for example, the nucleotide sequence identity of 60% or less, preferably exhibits the nucleotide sequence identity of 55% or less, more preferably exhibits the nucleotide sequence identity of 50% or less, still more preferably exhibits the nucleotide sequence identity of 45% or less, and particularly preferably exhibits the nucleotide sequence identity of 40% or less, 35% or less or 30% or less to the nucleotide sequence of the single strand polynucleotide portion in the nucleic acid probe for detection. In a particularly preferred embodiment, the nucleotide sequence of the single strand polynucleotide portion in the nucleic acid probe for detection exhibits the nucleotide sequence identity of 100% to the nucleotide sequence of a specific region in the complementary strand of the target nucleic acid, and the nucleotide sequence of the single strand polynucleotide portion in the nucleic acid probe for correction exhibits the aforementioned nucleotide sequence identity to the nucleotide sequence of the single strand polynucleotide portion in the nucleic acid probe for detection. When the nucleotide sequence of the single strand polynucleotide portion in the nucleic acid probe for correction and the nucleotide sequence of the single strand polynucleotide portion in the nucleic acid probe for detection are different in length each other, the nucleotide sequence percent identity is calculated based on the length of the longer nucleotide sequence. The nucleotide sequence percent identity can be determined by a method well-known in the art, and for example, may be determined as described above.

The nucleic acid probe for correction can also be used as a versatile probe for samples derived from any animals such as human. For example, it is known that human genome is composed of approximately 3,000,000,000 nucleotides. Theoretically, if a polynucleotide has 16 nucleotide residues in length, one locus specific for the human genome can be defined (4¹⁶=about 4,300,000,000). In other words, if a polynucleotide has 17 or more nucleotide residues in length, a single strand polynucleotide that is completely uncomplementary to any region on the human genome may be designed. Therefore, the single strand polynucleotide portion in the nucleic acid probe for correction may be uncomplementary to any region on the human genome when having the nucleotide length as described in the previous paragraph. The versatile nucleic acid probe for correction, which is uncomplementary to any region on the human genome and any region on optional human RNA (e.g., mRNA, microRNA) can be designed by checking a candidate nucleotide sequence in human gene information (e.g., genome, cDNA, RNA) database, taking a nucleotide sequence that exhibited a high identity to the candidate nucleotide sequence from the database, then again checking a candidate nucleotide sequence modified to exhibit a lower identity to the nucleotide sequence in the data base, and repeating these operations. The data can also be checked in gene information database for viruses and microorganisms that cause infectious diseases. The nucleic acid probe for correction having the single strand polynucleotide portion designed by such a technique is useful as a versatile probe for human-derived samples, and is used suitably for diagnoses and therapeutic monitoring in human.

The detection signal generation portion in the nucleic acid probe for correction is composed of a substance capable of generating a detectable signal, and enables to correct a detection signal from a subject to be measured in the isothermal nucleic acid amplification reaction. Example of the detection signal generation portion may include fluorescent substances.

When the detection signal generation portion is the fluorescent substance, the nucleic acid probe for correction may be a probe taking advantage of a fluorescence quenching phenomenon by a nucleobase. Such a nucleic acid probe for correction comprises the above single strand polynucleotide portion and the fluorescent substance having a nature that fluorescence is quenched by formation of a double strand nucleic acid structure. Examples of the fluorescent substance having the nature that fluorescence is quenched by formation of the double strand nucleic acid structure may include fluorescent substances in which a fluorescence signal reduces when positioned adjacent to the guanine base. Examples of the fluorescent substance in which a fluorescence signal reduces when positioned adjacent to a guanine base may include, for example, those described above. When the nucleic acid probe for detection is the probe taking advantage of the fluorescence quenching phenomenon by the nucleobase, it is preferable that the nucleic acid probe for correction is also the probe taking advantage of the fluorescence quenching phenomenon by the nucleobase because the nucleic acid probe for correction mimics the behavior of the nucleic acid probe for detection in the isothermal nucleic acid amplification reaction.

When the detection signal generation portion is the fluorescent substance, the nucleic acid probe for correction may also be the probe that does not emit fluorescence in an ordinary state. Such a nucleic acid probe for correction further comprises a quencher in addition to the above single strand polynucleotide portion and the fluorescent substance, and emits fluorescence when the fluorescent substance is separated from the quencher in distance. Examples of such a probe may include the hydrolysis probe and the stem-loop structured probe as described above. Examples of the fluorescent substance and the quencher used for such a nucleic acid probe for correction may include those described above for the nucleic acid probe for detection. When the nucleic acid probe for detection is the probe that does not emit the fluorescence in the ordinary state, it is preferable that the nucleic acid probe for correction is also the probe that does not emit the fluorescence in the ordinary state because the nucleic acid probe for correction mimics the behavior of the nucleic acid probe for detection in the isothermal nucleic acid amplification reaction. In particular, it is preferable that when the nucleic acid probe for detection is the hydrolysis probe, the nucleic acid probe for correction is also the hydrolysis probe, and it is preferable that when the nucleic acid probe for detection is the stem-loop structured probe, the nucleic acid probe for correction is also the stem-loop structured probe.

In the method of the present invention, a single nucleic acid probe for correction or a plurality (e.g., 2, 3, or 4) of nucleic acid probes for correction may be employed. When a plurality of nucleic acid probes for correction is employed, each of a plurality of nucleic acid probes for correction may comprise different detection signal generation portions. It is also preferable to comprise the same detection signal generation portion. When a plurality of nucleic acid probes for correction comprises the same detection signal generation portion, detection signals from the nucleic acid probes for correction can be averaged to utilize for correction of a detection signal from a subject to be measured.

In a preferred embodiment, both the detection signal generation portion in the nucleic acid probe for correction and the detection signal generation portion in the nucleic acid probe for detection is the fluorescent substance in which a fluorescence signal reduces when positioned adjacent to the guanine base. In this case, a nucleotide residue at a 3' end or a 5' end of the single strand polynucleotide portion in the nucleic acid probe for correction and the nucleic acid probe for detection is a cytosine-containing nucleotide, and is linked to the fluorescent substance in which a fluorescence signal reduces when the cytosine-containing nucleotide is positioned adjacent to the guanine base. When the nucleic acid probe for detection has the cytosine-containing nucleotide linked to the fluorescent substance in which a fluorescence signal reduces when the cytosine-containing nucleotide is positioned adjacent to the guanine base, at the 3' end or the 5' end of the single strand polynucleotide portion thereof, the nucleic acid probe for detection is complementarily bound to the target nucleic acid, thereby the fluorescent substance is positioned adjacent to the guanine base to quench a fluorescence signal from the fluorescent substance due to the effect of the guanine base. The nucleic acid probe for correction mimics the behavior of the nucleic acid probe for detection in the isothermal nucleic acid amplification reaction. Thus, it is preferable that a constitution of the nucleic acid probe for correction (end labeling pattern of the nucleic acid probe for correction with the fluorescent substance in which a fluorescence signal reduces when positioned adjacent to the guanine base) is the same as that of the nucleic acid probe for detection. Specifically, it is preferable to have any one of the following constitution of (I) or (II):
(I) the nucleic acid probe for correction has a cytosine-containing nucleotide linked to a first fluorescent substance in which a fluorescence signal reduces when positioned adjacent to the guanine base, at the 3' end of the single strand polynucleotide portion thereof, and the nucleic acid probe for detection has a cytosine-containing nucleotide linked to a second fluorescent substance in which a fluorescence signal reduces when positioned adjacent to the guanine base, at the 3' end of the single strand polynucleotide portion thereof; or
(II) the nucleic acid probe for correction has a cytosine-containing nucleotide linked to a first fluorescent substance in which a fluorescence signal reduces when positioned adjacent to the guanine base, at the 5' end of the single strand polynucleotide portion thereof, and the nucleic acid probe for detection has a cytosine-containing nucleotide linked to a second fluorescent substance in which a fluorescence signal reduces when positioned adjacent to the guanine base, at the 5' end of the single strand polynucleotide portion thereof.

In the case of (II), it is preferable that the 3' end of the single strand polynucleotide portion is dephosphorylated in order to avoid an extension reaction by a polymerase.

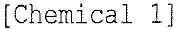

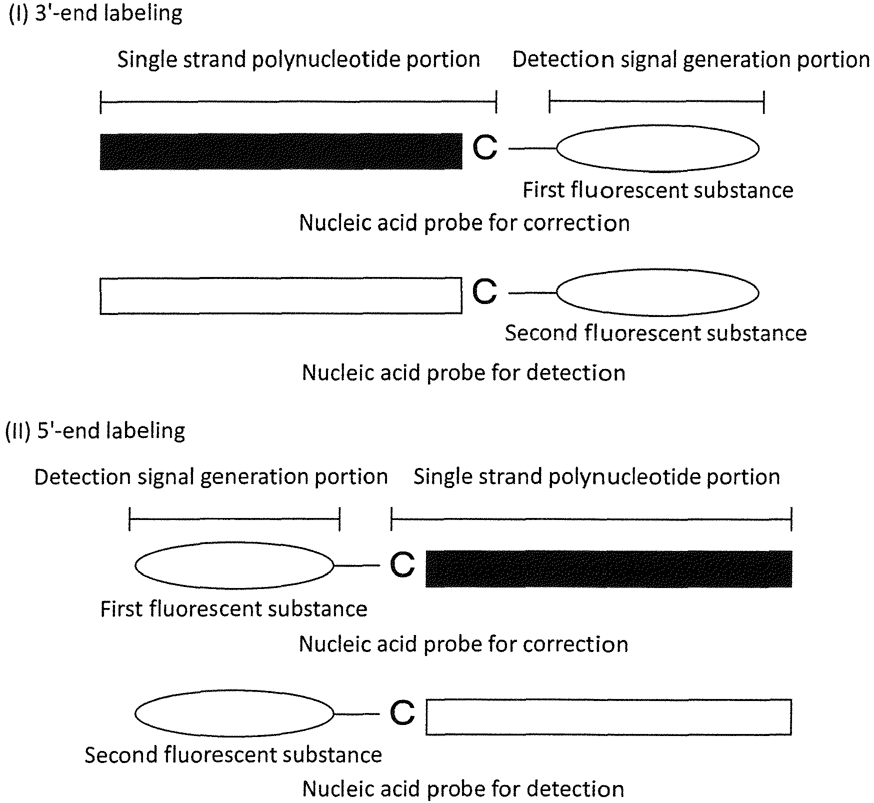

A nucleic acid sample itself such as DNA or RNA, or any sample containing the same is used as a nucleic acid sample. The nucleic acid sample is not particularly limited, and may be a sample containing or suspected of containing a nucleic acid component derived from an animal such as a mammal, a plant, an insect, a microorganism (e.g., bacterium, yeast), a virus (e.g., hepatitis virus such as HAV, HBV or HCV, or retrovirus such as HIV). Examples of the mammal may include primates (e.g., human, monkey, chimpanzee), rodents (e.g., mouse, rat, guinea pig), pets (e.g., dog. cat, rabbit), and working animals or domestic animals (e.g., cattle, horse, pig, sheep, goat). The nucleic acid sample may be a biological sample (e.g., blood, saliva, hair, mucosa, and tissue samples) derived from a mammal affected or suspected of being affected with a disease. The nucleic acid sample is used for the method of the present invention after extracting the nucleic acid as needed. When the subject to be measured is RNA, one obtained after a reverse transcription reaction of RNA can be used for the method of the present invention.

In the present invention, the nucleic acid sample is subjected to the amplification reaction of the target nucleic acid under the isothermal condition in the presence of the nucleic acid probe for correction and the nucleic acid probe for detection of the target nucleic acid as described above. The amplification reaction of the target nucleic acid under the isothermal condition is appropriately performed according to a reaction condition of the isothermal nucleic acid amplification reaction to be employed.

For example, a reaction temperature is the same as the temperature aforementioned in the isothermal nucleic acid amplification reaction. Specifically, the isothermal nucleic acid amplification reaction may be performed under the condition at high temperature of 50°C or higher (e.g., 50°C or higher and lower than 70°C) or under the condition at low temperature of lower than 50°C (e.g., 25°C or higher and lower than 50°C). When the isothermal nucleic acid amplification reaction is performed under the condition at low temperature, the temperature may be lower than 50°C, preferably lower than 45°C, and more preferably lower than 42°C.

A concentration of the nucleic acid probe for detection in a reaction solution used for the isothermal nucleic acid amplification reaction is, for example, 0.01 to 2 µM, preferably 0.02 to 1 µM, and more preferably 0.04 to 0.5 µM. A concentration of the nucleic acid probe for correction in the reaction solution is, for example, 0.01 to 2 µM, preferably 0.02 to 1 µM, and more preferably 0.04 to 0.5 µM.

These concentrations are also preferably close each other because the nucleic acid probe for correction mimics the behavior of the nucleic acid probe for detection in the isothermal nucleic acid amplification reaction. Therefore, a ratio of the concentration of the nucleic acid probe for correction to the concentration of the nucleic acid probe for detection (the nucleic acid probe for detection/the nucleic acid probe for correction) is, for example, within the range of 1/100 to 100, preferably within the range of 1/10 to 10, more preferably within the range of 1/5 to 5, still more preferably within the range of 1/2 to 2, and particularly preferably 1 (i.e., equal concentration).

Other conditions (e.g., primer concentrations, an amount of the nucleic acids added to the reaction solution, a reaction time period) in the isothermal nucleic acid amplification reaction can be appropriately determined.

The correction of the detection signal due to the nucleic acid probe for detection can be performed with the detection signal due to the nucleic acid probe for correction according to a publicly known method concerning the correction of the detection signal. For example, the detection signal due to the nucleic acid probe for detection can be corrected with dividing the detection signal due to the nucleic acid probe for detection by the detection signal due to the nucleic acid probe for correction.

The present invention also provides a kit.

The kit of the present invention comprises a nucleic acid probe for correction and a nucleic acid probe for detection of the target nucleic acid. Details of the nucleic acid probe for correction and the nucleic acid probe for detection of the target nucleic acid are as described above. The kit of the present invention is used, for example, for the aforementioned nucleic acid amplification reaction.

The kit of the present invention may comprise a polymerase. Examples of the polymerase may include a DNA polymerase and an RNA polymerase.

When the polymerase is the DNA polymerase, the kit of the present invention may further comprise one or more enzymes selected from the group consisting of a restriction enzyme, a ribonuclease H, a ligase, a helicase and a recombinase. In this case, the kit of the present invention may comprise a melting temperature adjuster (e.g., betaine) or a single strand DNA binding protein (SSB) in addition to the above enzyme. In this case, the kit of the present invention may further comprise dNTPs. The kit of the present invention may further comprise ATP when comprising the recombinase.

When the polymerase is the RNA polymerase, the kit of the present invention may further comprise a reverse transcriptase (e.g., an enzyme having an RNase H activity or an enzyme having no RNase H activity). In this case, the kit of the present invention may comprise either one of dNTPs or NTPs, preferably both of dNTPs and NTPs.

The kit of the present invention may comprise primers for the target nucleic acid. One or more primers are included in the kit of the present invention, and the number of the primers can vary (e.g., 1, 2, 3 or 6) depending on the nucleic acid amplification reaction to be employed. Examples of the primer may include a DNA or RNA molecule, and a hybrid molecule of DNA and RNA.

Specifically, the kit of the present invention may also comprise the components as described in Table 1 depending on the nucleic acid amplification reaction to be employed. The kit of the present invention may also comprise a positive control [e.g., a normal or abnormal target nucleic acid (e.g., DNA, RNA)] and/or a negative control [e.g., an abnormal or normal target nucleic acid (e.g., DNA, RNA)]. The kit of the present invention may further comprise another control such as a nucleic acid (e.g., DNA, RNA) for a housekeeping gene (e.g., GAPDH, β-actin, β2-microglobulin, HPRT1) that can be used as a control for comparison in a qualitative and/or quantitative assay. The kit of the present invention may also comprise the primers for these controls. In the kit of the present invention, each component in an isolated form may be included in an individual container (e.g., a tube), or two or more components may be previously mixed in the same container.

**[Table 1]**

| Table 1. Components in kit | | | | |
|---|---|---|---|---|
| | Essential enzyme | Preferable component | Number of primers | Other component |
| Method mediated by transcription | | | | |
| TMA | Reverse transcriptase having RNase H activity | | 2 | I dNTPs |
| | | | | NTPs |
| | RNA polymerase | | | |
| NASBA | Reverse transcriptase | | 2 | dNTPs |
| | RNA polymerase Ribonuclease H | | | NTPs |
| | | | | |

| Method using a nicking enzyme | | | | |
|---|---|---|---|---|
| SDA | DNA polymerase | | 2 types | dNTPs |
| | Restriction enzymes | | | |
| ICAN | DNA polymerase | | 2 (hybrid molecule of DNA and RNA) | dNTPs |
| | Ribonuclease H | | | |
| | | | | |

| Method utilizing a series of DNA synthesis through loop structure | | | | |
|---|---|---|---|---|
| LAMP | DNA polymerase | Melting temperature adjuster (e.g., betaine) | 6 | dNTPs |
| | | | | |

| Method utilizing rolling circle type of DNA replication reaction | | | | |
|---|---|---|---|---|
| RCA | DNA polymerase | | 1 | dNTPs |
| | Ligase | | | Padlock probe |
| | | | | |

| Method using a helicase | | | | |
|---|---|---|---|---|
| HDA | DNA polymerase Helicase | SSB | 2 | dNTPs |
| | | | | |

| Method using a recombinase | | | | |
|---|---|---|---|---|
| RPA | DNA polymerase | SSB | 2 | dNTPs |
| | Recombinase | | | ATP |
| SIBA | DNA polymerase | SSB | 3 | dNTPs |
| | Recombinase | | | ATP |

### EXAMPLES

The present invention will be described with reference to following Examples, but the present invention is not limited the following Examples.

### (Materials)

The nucleic acid probe for detection consisting of the nucleotide sequence shown in Table 2 and a nucleic acid probe for correction consisting of a nucleotide sequence shown in Table 3 were used in the following experiments.

**[Table 2]**

| Table 2. Nucleotide sequence of nucleic acid probe for detection | | |
|---|---|---|
| Name | Nucleotide sequence | SEQ ID NO |
| HB_detection Probe | ttc ctc ttc atc ctg ctg cta tgc ctc atc ttc | SEQ ID NO:1 |

**[Table 3]**

| Table 3. Nuccleotide sequence of nucleic acid probe for correction | | |
|---|---|---|
| Name | Nucleotide sequence | SEQ ID NO |
| Reference-1 | ttt ggt gaa gag ttt tat ggc gtc | SEQ ID NO:2 |
| Reference-2 | ctc ttt ggt gaa gag ttt tat ggc gtc | SEQ ID NO:3 |
| Reference-3 | ggg ctc ttt ggt gaa gag ttt tat ggc gtc | SEQ ID NO:4 |
| Reference-4 | tag ggg ctc ttt ggt gaa gag ttt tat ggc gtc | SEQ ID NO:5 |
| Reference-5 | tgt tag ggg ctc ttt ggt gaa gag ttt tat ggc gtc | SEQ ID NO:6 |
| Reference-6 | gtg tgt tag ggg ctc ttt ggt gaa gag ttt tat ggc gtc | SEQ ID NO:7 |
| Reference-7 | tgt gtg tgt tag ggg ctc ttt ggt gaa gag ttt tat ggc gtc | SEQ ID NO:8 |

Details of the nucleic acid probe for detection and the nucleic acid probe for correction are as shown in Tables 4 and 5, respectively.

**[Table 4]**

| Table 4. Details of nucleic acid probe for detection | | | | |
|---|---|---|---|---|
| Name | Tm(°C) | GC (%) | Length | Label at 3' end |
| HB_detection Probe | 68.18 | 48.5% | 33 | BODIPY |

**[Table 5]**

| Table 5. Details of nucleic acid probe for correction | | | | |
|---|---|---|---|---|
| Name | Tm (°C) | GC (%) | Length | Label at 3' end |
| Reference-1 | 58.82 | 41.7% | 24 | TAMRA |
| Reference-2 | 61.40 | 44.4% | 27 | TAMRA |
| Reference-3 | 66.68 | 50.0% | 30 | TAMRA |
| Reference-4 | 68.56 | 48.5% | 33 | TAMRA |
| Reference-5 | 69.85 | 47.2% | 36 | TAMRA |
| Reference-6 | 71.14 | 48.7% | 39 | TAMRA |
| Reference-7 | 72.78 | 47.6% | 42 | TAMRA |

### (Example 1)

TwistAmp Basic kit supplied from TwistDx utilizing an RPA method as a principle was used for a nucleic acid amplification assay. This kit comprises a DNA polymerase, a recombinase, dNTPs, ATP and a single strand DNA binding protein (SSB) as components required for a nucleic acid amplification reaction. Therefore, these components were used in assays shown below. Two primers for amplifying the nucleic acid were artificially synthesized by Sigma, the target nucleic acid was artificially synthesized by Invitrogen, and the nucleic acid probe for detection (HB_detection probe) and the nucleic acid probe for correction (Reference-1) were artificially synthesized by Nittestu Kankyo Engineering K.K. These were used.

A forward primer: 5'-gctatcgctggatgtgtctgcggcgttttatca-3' (SEQ ID NO:9) and a reverse primer: 5'-ttgcgaaagcccaggatgatgggatgggaatac-3' (SEQ ID NO:10) were used as the sequences of two primers. A gene sequence corresponding to an S antigen site of HBV was used as the target nucleic acid. The nucleic acid probe for detection was labeled with BODIPY at its 3' end, and the nucleic acid probe for correction was labeled with TAMRA at its 3' end.

Rehydration buffer containing 0.2 µM of the forward primer, 0.4 µM of the reverse primer, 0.05 µM of the nucleic acid probe for detection, and 0.05 µM of the nucleic acid probe for correction was prepared. Subsequently, 1,000 copies/0.05 µL of the target nucleic acid was added, and then 47.5 µL of the prepared solution was added to an RPA pellet and the mixture was dissolved. Likewise, a solution comprising distilled water (DW) in place of the target nucleic acid was prepared.

To the solution prepared above, 2.5 µL of 280 mM magnesium acetate was added, and the mixture was stirred well and incubated at 37°C for 30 minutes. During the incubation, fluorescence signals were measured every 30 seconds.

The obtained data were processed as follows. After calculating Fluorescence intensity ratio at each measurement time [=Fluorescence intensity signal from nucleic acid probe for detection/Fluorescence intensity signal from nucleic acid probe for correction], change rate of the fluorescence intensity ratio at each measurement time (fluorescence intensity change rate) based on the fluorescence intensity ratio 5 minutes after starting the reaction was obtained (FIG. 1). The similar experiment was performed in the absence of the nucleic acid probe for correction (not corrected), and an experimental result obtained when the data had not been corrected was compared with an experimental result obtained when the data had been corrected with the nucleic acid probe for correction (FIG. 1).

As a result, the fluorescence intensity change rate on a baseline of the detection signal ("DW" in FIG. 1) was stabilized in the presence of the nucleic acid probe for correction (Reference-1 that is a nucleic acid probe). Specifically, in an early phase of a reaction process, the fluorescence intensity change rate on the baseline (1 minute) exhibited a low value (1.15%) when corrected with the nucleic acid probe for correction (FIG. 1). The fluorescence intensity change rate on the baseline was not stabilized in the early phase of the reaction process when not corrected. On the other hand, it was stabilized in a short period of time (2 minutes) when corrected with the nucleic acid probe for correction (FIG. 1). The fluorescence intensity change rate on the baseline was confirmed to be stabilized in a late phase of the reaction process when corrected with the nucleic acid probe for correction (FIG. 1). The stabilization of the fluorescence intensity change rate on the baseline of the detection signals contributes to stable correction of the detection signal from the subject to be measured in the isothermal nucleic acid amplification reaction ("10³ copies/assay" in FIG. 1). Therefore, it was demonstrated that a nucleic acid probe for correction was beneficial for correction of detection signals from a subject to be measured in an isothermal nucleic acid amplification reaction.

### (Reference Example 1)

An experiment was performed to obtain a fluorescence intensity change rate in the same manner as in Example 1, except that ROX reference dye supplied from Invitrogen was used in place of the nucleic acid probe for correction (FIG. 2). A similar experiment was performed in the absence of ROX (not corrected), and an experimental result obtained when the data had not been corrected was compared with an experimental result obtained when the data had been corrected with ROX (FIG. 2).

As a result, the fluorescence intensity change rate on the baseline could not been stabilized by ROX (non-nucleic acid molecule) in the isothermal nucleic acid amplification reaction (FIG. 2). That is, it is thought that ROX is useful as control in accuracy management of experimental manipulations (e.g., dispensing of reagents for preparing the reaction solution), but it was demonstrated that ROX was not always useful for the correction of the detection signal from the subject to be measured in the isothermal nucleic acid amplification reaction.

### (Example 2) Investigation for length of nucleic acid probe for correction

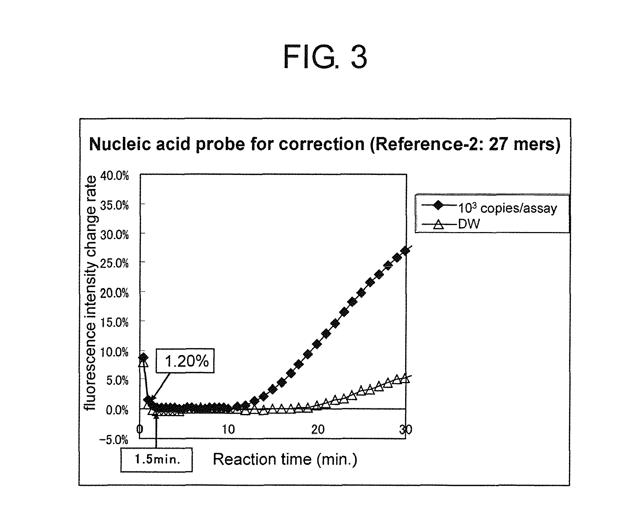

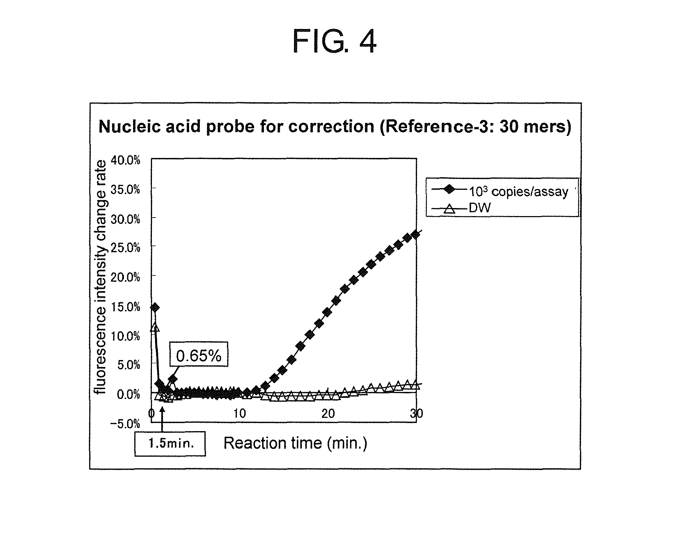

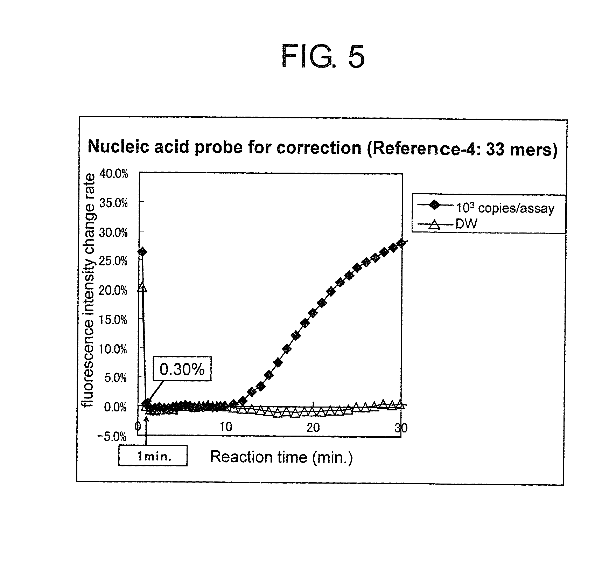

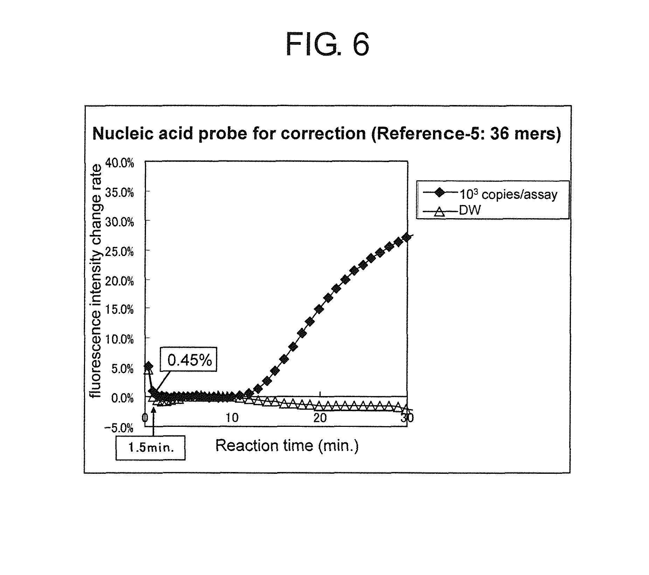

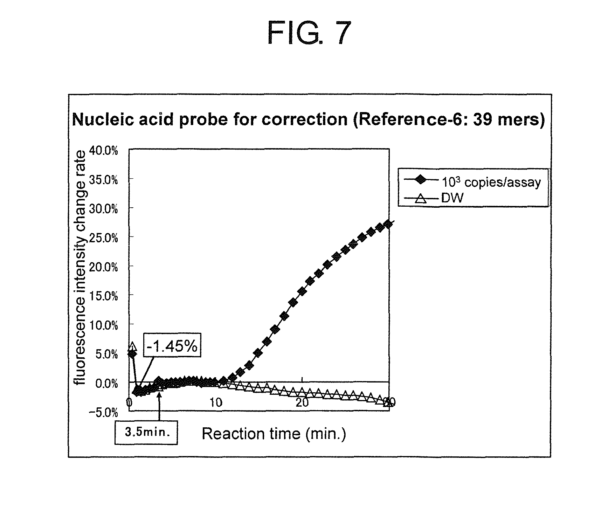

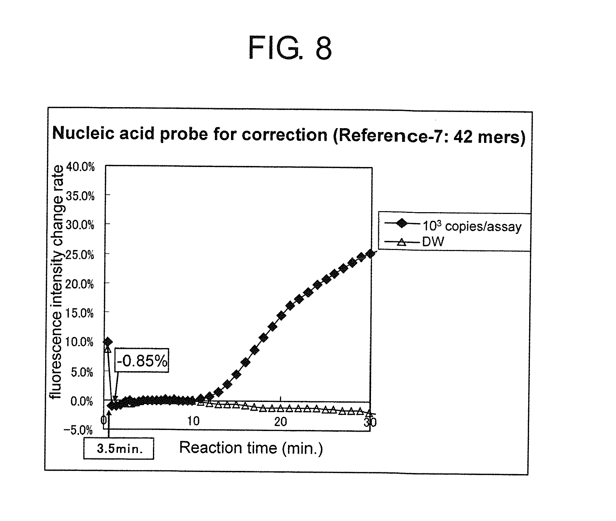

Experiments were performed to obtain the fluorescence intensity change rates in the same manner as in Example 1, except that a nucleic acid probe for correction that is different in length (Reference 2: 27 bases, Reference 3: 30 bases, Reference 4: 33 bases, Reference 5: 36 bases, Reference 6: 39 bases, or Reference 7: 42 bases) was used in place of Reference-1 (24 bases) (FIGs. 3 to 8). Experimental results in this Example together with the experimental results in Example 1 and Reference Example 1 were summarized as follows in Tables 6 and 7.

**[Table 6]**

| Table 6. Stabilization of baseline in early phase of reaction process | | |
|---|---|---|
| | Fluorescence change rat e on baseline (1 min.) | Time required for stabiliza tion of baseline (min.) |
| ROX | 7.60% | 4.5 |
| Reference-1 | 1.15% | 2.0 |
| Reference-2 | 1.20% | 1.5 |
| Reference-3 | 0.65% | 1.5 |
| Reference-4 | 0.30% | 1.0 |
| Reference-5 | 0.45% | 1.5 |
| Reference-6 | -1.45% | 3.5 |
| Reference-7 | -0.85% | 3.5 |

**[Table 7]**

| Table 7. Change on baseline in late phase of reaction process | | |
|---|---|---|
| | Fluorescence change rate on baseline (20 min) | Fluorescence change rate on baseline (30 min) |
| ROX | 4.70% | 8.80% |
| Reference-1 | -1.10% | -2.60% |
| Reference-2 | 0.60% | 5.30% |
| Reference-3 | -0.30% | 1.50% |
| Reference-4 | -0.80% | 0.50% |
| Reference-5 | -1.50% | -2.10% |
| Reference-6 | -1.70% | -3.50% |
| Reference-7 | -1.20% | -1.90% |

It was confirmed from Tables 6 and 7 that the nucleic acid probes for correction (References-1 to 8, nucleic acid probes) were more excellent than ROX (non-nucleic acid probe) in stabilization of the fluorescence intensity change rate on the baseline (i.e., correction of detection signals in an isothermal nucleic acid amplification reaction). It was also confirmed that the effect of the nucleic acid probe for correction was particularly excellent when the difference in length between the nucleic acid probe for correction and the nucleic acid probe for detection was within 3 nucleotide residues.

### INDUSTRIAL APPLICABILITY

The present invention is useful for measurement of a target nucleic acid by an isothermal nucleic acid amplification reaction.

## Claims

1. A method for detecting a target nucleic acid in an isothermal nucleic acid amplification reaction, comprising the following (1) and (2):
(1) subjecting a nucleic acid sample to an amplification reaction of a target nucleic acid under an isothermal condition in the presence of a nucleic acid probe for correction and a nucleic acid probe for detection of the target nucleic acid; and
(2) correcting a detection signal due to the nucleic acid probe for detection with a detection signal due to the nucleic acid probe for correction in the amplification reaction.

2. The method according to claim 1, wherein the nucleic acid probe for correction comprises a single strand polynucleotide portion comprising a nucleotide sequence that is not complementary to a nucleotide sequence of the target nucleic acid, a detection signal generation portion,
wherein the nucleic acid probe for detection comprises a single strand polynucleotide portion comprising a nucleotide sequence that is complementary to the nucleotide sequence of the target nucleic acid, and a detection signal generation portion,
wherein the detection signal generation portion of the nucleic acid probe for correction and the detection signal generation portion of the nucleic acid probe for detection are different from each other.

3. The method according to claim 2, wherein the nucleic acid probe for correction comprises a single strand polynucleotide portion consisting of 8 to 50 nucleotide residues,
wherein the nucleic acid probe for detection comprises a single strand polynucleotide portion consisting of 8 to 50 nucleotide residues.

4. The method according to claim 3, wherein a difference in length between the nucleic acid probe for correction and the nucleic acid probe for detection is within the range of 9 nucleotide residues.

5. The method according to claim 4, wherein the difference in length between the nucleic acid probe for correction and the nucleic acid probe for detection is within the range of 3 nucleotide residues.

6. The method according to any one of claims 2 to 5, wherein the nucleotide sequence of the single strand polynucleotide portion in the nucleic acid probe for correction exhibits 50% or less identity to the nucleotide sequence of the single strand polynucleotide portion in the nucleic acid probe for detection.

7. The method according to claim 2, wherein the detection signal generation portion in the nucleic acid probe for correction and the detection signal generation portion in the nucleic acid probe for detection are different fluorescent substances.

8. The method according to claim 7, wherein the fluorescent substance has a nature that fluorescence is quenched by formation of a double strand nucleic acid structure.

9. The method according to claim 8, which is either the following (I) or (II):
(I) the nucleic acid probe for correction has a cytosine-containing nucleotide linked to a first fluorescent substance in which a fluorescence signal reduces when positioned adjacent to a guanine base, at a 3' end of the single strand polynucleotide portion thereof, and the nucleic acid probe for detection has a cytosine-containing nucleotide linked to a second fluorescent substance in which a fluorescence signal reduces when positioned adjacent to a guanine base, at a 3' end of the single strand polynucleotide portion thereof; or
(II) the nucleic acid probe for correction has a cytosine-containing nucleotide linked to a first fluorescent substance in which a fluorescence signal reduces when positioned adjacent to a guanine base, at a 5' end of the single strand polynucleotide portion thereof, and the nucleic acid probe for detection has a cytosine-containing nucleotide linked to a second fluorescent substance in which a fluorescence signal reduces when positioned adjacent to a guanine base, at a 5' end of the single strand polynucleotide portion thereof.

10. The method according to any one of claims 1 to 9, wherein the target nucleic acid is amplified under an isothermal condition at lower than 50°C.

11. The method according to any one of claims 1 to 10, wherein the isothermal nucleic acid amplification reaction is a method using a recombinase.

12. A kit comprising a nucleic acid probe for correction and a nucleic acid probe for detecting a target nucleic acid.

13. The kit according to claim 12, further comprising a polymerase.

14. The Kit according to claim 12 or 13, further comprising a recombinase.


SEQUENCE LISTING

<110>  FUJIREBIO INC.

<120>  Methof of calibrating isothermal nucleic acid-amplifying reaction
        under isothermal condition using nucleic acid probe

<130>  PRBA-25008

<150>  JP2012-018277
<151>  2012-01-31

<160>  10

<170>  PatentIn version 3.5

<210>  1
<211>  33
<212>  DNA
<213>  Artificial Sequence

<220>
<223>  HB_detection Probe

<400>  1
ttcctcttca tcctgctgct atgcctcatc ttc                                    33


<210>  2
<211>  24
<212>  DNA
<213>  Artificial Sequence

<220>
<223>  Reference-1

<400>  2
tttggtgaag agttttatgg cgtc                                              24


<210>  3
<211>  27
<212>  DNA
<213>  Artificial Sequence

<220>
<223>  Reference-2

<400>  3
ctctttggtg aagagtttta tggcgtc                                           27


<210>  4
<211>  30
<212>  DNA
<213>  Artificial Sequence

<220>
<223>  Reference-3

<400>  4
gggctctttg gtgaagagtt ttatggcgtc                                        30

<210>  5
<211>  33
<212>  DNA
<213>  Artificial Sequence

<220>
<223>  Reference-4

<400>  5
taggggctct ttggtgaaga gttttatggc gtc                                    33


<210>  6
<211>  36
<212>  DNA
<213>  Artificial Sequence

<220>
<223>  Reference-5

<400>  6
tgttaggggc tctttggtga agagttttat ggcgtc                                 36


<210>  7
<211>  39
<212>  DNA
<213>  Artificial Sequence

<220>
<223>  Reference-6

<400>  7
gtgtgttagg ggctctttgg tgaagagttt tatggcgtc                              39


<210>  8
<211>  42
<212>  DNA
<213>  Artificial Sequence

<220>
<223>  Reference-7

<400>  8
tgtgtgtgtt aggggctctt tggtgaagag ttttatggcg tc                          42


<210>  9
<211>  33
<212>  DNA
<213>  Artificial Sequence

<220>
<223>  Forward primer for amplifying a polynucleitide encoding S antigen
       of HBV

<400>  9
gctatcgctg gatgtgtctg cggcgtttta tca                                    33


<210>  10

<211>  33
<212>  DNA
<213>  Artificial Sequence

<220>
<223>  Reverse primer for amplifying a polynucleitide encoding S antigen
       of HBV

<400>  10
ttgcgaaagc ccaggatgat gggatgggaa tac                                    33